# EUROPEAN PATENT APPLICATION

(11) **EP 3 520 818 A1**
(43) Date of publication of application: **07.08.2019**
(21) Application number: 18154582.3
(22) Date of filing: 01.02.2018
(51) Int. Cl.: A61K 45/06, A61K 31/045, A61K 31/05, A61K 31/352, A61K 31/355, A61K 31/375, A61P 17/16, A61P 17/02, A61P 17/00, A61P 43/00, A61K 9/00

(54) **COMPOSITIONS FOR TREATING AND /OR PREVENTING PHOTODYNAMIC THERAPY SIDE EFFECTS**

(71) Applicant: AC BioScience SA, 1024 Ecublens (CH)
(72) Inventor: AUCLAIR, Christian, 78730 Saint-Arnoult en Yvelines (FR)
(74) Representative: KATZAROV S.A.

(57) **Abstract**

The present invention relates to a composition combination for treating and /or preventing Photodynamic therapy side effects.

## Description

### FIELD OF THE INVENTION

The present invention relates to a composition combination for treating and /or preventing Photodynamic therapy side effects.

### BACKGROUND OF THE INVENTION

Photodynamic therapy (PDT) is often chosen for treating early stages of local, superficial cancers in hollow organs and other regions of the body accessible to light application. PDT is also considered as the treatment of choice for, among others, actinic keratosis (in dermatology), and wet age-related macular degeneration (in ophthalmology). Some other use of PDT has been as well proposed such as the hair regrowth inhibition following depilation. To obtain the desired cytotoxic and healing effects, PDT relies on the presence in the target tissue of a photosensitizer (PS), molecular oxygen (O2), and administration of light at wavelengths absorbed by the PS. Photodynamic therapy is an interesting therapeutic option, but it presents drawbacks pain, lesion recurrences, hyperpigmentation and despite encouraging results, some clinicians avoid using this therapy.

As stated above, PDT treatment requires a photosensitizer (PS). In dermatology, the PS of choice is the group of molecules called photo-activable porphyrins (PaPs), the most widely studied member of this group being protoporphyrin IX (PpIX). Classical PDT procedure relies on the administration of a precursor of PpIX, Metvix ® (approved for AK and basal cell carcinoma in Europe) or Levulan ® (approved for AK in the USA). Metvix protocol consists in the topical administration of 5-aminolevulinic acid (ALA) leading, after a suitable drug-light interval, to biosynthesis and accumulation of photoactivable porphyrins (PAPs), including protoporphyrin IX (PpIX), the actual PS in the cells of the target tissue.

Topical photodynamic therapy (PDT) uses a broadband red-light source ranging between 570-670 nm. The illumination of the skin lesion with light of the appropriate wavelength results in the production of reactive oxygen species (ROS) including singlet oxygen. This process is toxic to targeted malignant and other diseased cells, and it has proven ability to kill microbial cells, including bacteria, fungi and viruses (warts and molluscum contagiosum). It is used clinically to treat a wide range of medical or cosmetic conditions, including acne, skin aging, psoriasis, granuloma annulare, age-related macular degeneration and malignant cancers, CTCL, and extramammary Paget disease and is recognized as a treatment strategy which is both minimally invasive and minimally toxic. The combination of photosensitizer, a light source and tissue oxygen, leads to the chemical destruction of any tissues which have either selectively taken up the photosensitizer or have been locally exposed to light, with recruitment of inflammatory cells, increased immune response, and vascular compromise. Single oxygen can also destroy photosensitizing agent itself preventing further action, a process referred to as photo-bleaching. The wavelength of the light source needs to be appropriate for exciting the photosensitizer to produce reactive oxygen species. These reactive oxygen species generated through PDT are free radicals (Type I PDT) generated through electron abstraction or transferred from a substrate molecule and highly reactive state of oxygen known as singlet oxygen (Type II PDT). In understanding the mechanism of PDT it is important to distinguish it from other light-based and laser therapies such as laser wound healing and rejuvenation, or intense pulsed light hair removal, which do not require a photosensitizer.

PDT is an improvement over other treatment in many applications, and especially in cosmetic applications, but it still suffers from some drawbacks. PDT displays various side effects including skin inflammation, pain and sometimes hyperpigmentation. This can be a serious limitation in the use of this technology especially when proposed for cosmetic purpose.

Although a variety of approaches for alleviating PDT-associated discomfort and pain have been attempted, there is still a need for topical treatment that prevents/minimizes side effects associated with the activation of the photosensitizer by irradiation.

The present invention addresses this need by providing a combination for use in the treatment and/or prevention of Photodynamic therapy side effects.

### SUMMARY OF THE INVENTION

This object has been achieved by providing a composition combination for use in the topical treatment and/or prevention of Photodynamic therapy side effect(s), said pharmaceutical combination comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
wherein said first and second pharmaceutical compositions are administered separately in a subject in need thereof

A further object of the present invention is to provide a kit comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
optionally with instructions to use.

A further object of the present invention is to provide a method for the treatment and/or prevention of Photodynamic therapy side effects comprising separately administering in a subject in need thereof
i) a first pharmaceutical composition comprising a therapeutically effective amount of one or more anti-inflammatory agent(s) and/or one or more oxy-radical scavenger(s),
ii) a second pharmaceutical composition comprising a therapeutically effective amount of one or more anti-inflammatory agent(s) and/or one or more anti-pigmentation compound(s),
wherein administration is a topical administration.

### BRIEF DESCRIPTION OF THE FIGURES

Figure 1. Inhibitory effect of DHQ on the reactive oxygen species (ROS) production resulting from the neutrophils activation by 100 ng/ml PMA.
Figure 2. Dihydroquercetin (DHQ) mediated inhibition of IL6 release from activated mast cells.
Figure 3. Effect of PHOTOOXYL protocol on the extent of PDT-induced skin inflammation (example VI).
Figure 4. Effect of PHOTOOXYL protocol on the extent of PDT-induced skin inflammation (example VII).

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

Reference throughout this specification to "one aspect" "an aspect" "another aspect," "a particular aspect," combinations thereof means that a particular feature, structure or characteristic described in connection with the invention aspect is included in at least one aspect of the present invention. Thus, the appearances of the foregoing phrases in various places throughout this specification are not necessarily all referring to the same aspect. Furthermore, the particular features, structures, or characteristics may be combined in any suitable manner in one or more aspects.

The term "comprise" or "comprising" is generally used in the sense of "include" or "including", that is to say, permitting the presence of one or more features or components. The terms "comprise" and "comprising" also encompass the more restricted ones "consist" and "consisting", respectively.

The term "about" indicates a defined range around that value. If "X" were the value, "about X" would generally indicate a value from 0.90X to 1.1 OX. Any reference to "about X" minimally indicates at least the values X, 0.90X, 0.9 IX, 0.92X, 0.93X, 0.94X, 0.95X, 0.96X, 0.97X, 0.98X, 0.99X, 1.01X, 1.02X, 1.03X, 1.04X, 1.05X, 1.06X, 1.07X, 1.08X, 1.09X, and 1.10X. Thus, "about X" is intended to disclose, e.g., "0.98X." When "about" is applied to the beginning of a numerical range, it applies to both ends of the range. Thus, "from about 6 to 8.5" or "from about 6-8.5" is equivalent to "from about 6 to about 8.5." When "about" is applied to the first value of a set of values, it applies to all values in that set. Thus, "about 7, 9, or 11%" is equivalent to "about 7%, about 9%, or about 11%."

As used in the specification and claims, the singular form "a", "an" and "the" include plural references unless the context clearly dictates otherwise.

As used herein, "one or more" compound(s)/agent(s) means "at least one" compound/agent, e.g. a combination of two, three, four, five, six, *etc...* compounds/agents.

Photodynamic therapy is currently used in a number of medical fields, including oncology, dermatology, ophthalmology, and oral medicine. PDT is also used for cosmetic purposes, e.g. for improving skin conditions selected among the group comprising acne, rosacea, depilation, sun damage, enlarged sebaceous glands, skin aging (wrinkles), warts, hidradenitis suppurativa, and psoriasis. The present invention can be effective in all the above-listed applications, whether in the cosmetic or in the medical field.

As discussed above, PDT treatment requires a photosensitizer (PS) such as those chosen from the group of molecules called photo-activable porphyrins (PaPs), the most widely studied member of this group being protoporphyrin IX (PpIX). Currently, the most commonly used precursors of protoporphyrin IX (PpIX) in dermatology are topical 5-ALA, methyl-ALA (MAL), and other intermediate photosensitizing porphyrins.

Topical photosensitizers are used mainly in the field of dermatology because they can be delivered directly to the skin and rarely cause prolonged phototoxicity. Topical PDT is a new and rapidly evolving therapeutic option for the treatment of inflammatory skin diseases (e.g. Acne vulgaris, Rosacea, Hidradenitis suppurativa, psoriasis, sarcoidosis), neoplastic skin diseases (Actinic keratosis, Squamous cell carcinoma, Basal cell carcinoma, Cutaneous T cell lymphoma, Kaposi's sarcoma, extramammary Paget's disease, cutaneous B cell lymphoma, and vascular malformations) and microbial skin diseases (e.g. Human papillomaviruses (HPV) warts, HPV cervical carcinoma, HPV anogenital SCC, and papillomatosis, verruca vulgaris, condyloma acuminatum, Epidermodysplasia verruciformis, Onychomycosis, Cutaneous leishmaniasis, verruca vulgaris, and condyloma acuminatum).

In the context of the present invention, the "subject" refers to a mammal, including dog, cat, rat, mouse, monkey, cow, horse, goat, sheep, pig, camel, and, most preferably, a human. The term "subject in need thereof" does not denote a particular age or sex. Thus, adult, infant and newborn subjects, whether male or female, are intended to be covered. Usually, the subject is a subject in need of PDT treatment, i.e. a subject undergoing, having undergone, or who will undergo, a PDT treatment.

As used herein, PDT treatment is not limited to the use of any particular type of light but also includes, e.g. natural daylight (natural daylight photodynamic therapy).

A "therapeutically effective amount" as used herein, means an amount of a compound or composition high enough to significantly positively modify the symptoms and/or condition to be treated, such as photodynamic therapy side effects. The therapeutically effective amount of the compounds of the invention is selected in accordance with a variety of factors including type, species, age, weight, sex and medical condition of the patient; the severity of the photodynamic therapy side effect or effects to be treated; and the route of administration. A physician of ordinary skill in the art can readily determine and prescribe the effective amount of the drug required to prevent, counter or arrest the progress of the condition. It is to be noted that throughout the specification (description and claims) and for the ease of reading, the term "therapeutically effective amount" refers to all the agents and compounds of the invention as well as to derivatives, active optical or steric isomers thereof.

"Administering" or "administration", as it applies herein, refers to contact of one or more pharmaceutical compositions of the invention, to the subject, preferably the subject in need thereof. In the context of the present invention, the administration of the pharmaceutical compositions of the combination composition is a topical administration to the skin.

Surprisingly, the inventors of the present invention have shown that a composition combination comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
can be used in the topical treatment and/or prevention of Photodynamic therapy side effects, in particular when said first and second pharmaceutical compositions are administered separately in a subject in need thereof.

Photodynamic therapy side effects refer to secondary, typically undesirable effects of PDT treatment and are selected among the non-limiting group comprising erythema, oedema, itching, epithelial exfoliation, pustules, inflammation, hyperpigmentation, and pain. Preferably, the side effects are selected from the group comprising inflammation, (hyper) pigmentation, and pain.

The major drawback of PDT treatment is pain. Most patients experience a burning sensation, also described as 'stinging' or 'prickling', in the ALA-treated area during light exposure. The mechanism for this reaction is not clear. A possible explanation is hyperthermia of the tissue; however, (Orenstein et al 1995) obtained performed IR imaging and could not relate the pain sensation with a temperature increase. This implies that the pain sensation is a consequence of the photochemical reaction in the tissue, and related to the presence of reactive singlet oxygen. This is consistent with the clinical experience that the peak pain is obtained after a few minutes of irradiation when the photodynamic activity is high, and then gradually decreased towards the background level (Kennedy and Pottier 1992). Some patients however also experience post-procedural pain in such a way that few patients interrupt treatment sessions.

The mechanism of pain associated with PDT is unknown but some theories have been presented. These include:
□ Stimulation or damage of the nociceptors in the epidermal nerve endings by hypoxia or by singlet oxygen produced during the irradiation.
□ Presence of inflammation mediators such as adenosine triphosphate (ATP) and bradykinin.
□ Stimulation of the transient receptor potential cation channel subfamily V member 1 (TRPV1), also known as the "capsaicin receptor" or the "vanilloid receptor". Stimulation of this receptor causes a lower temperature threshold which induces a burning, stinging pain at normal skin temperatures.
□ Another possible receptor involved in pain and pain relief by cooling is the transient receptor potential cation channel subfamily M member 8 (TRPM8) also known as the cold and menthol receptor. Membrane voltage might contribute to the fine turning of cold and heat sensitivity in sensory cells.

In fact, the occurrence of pain is clearly associated to PDT-induced skin inflammation. Experimental models show that PDT-induced reactive oxygen species initiate the synthesis and/or release of a range of mediators including prostanoids, nitric oxide, and histamine (Cecic and Korbelik, 2002), and upregulate the expression of several cytokines and chemokines, namely IL-1β, IL-2, IL-6, IL-8, IL-10, tumor necrosis factor-a, and G-CSF. Topical ALA-PDT employing red light penetrates deeply into the dermis and consequently may mediate its effects directly via both dermal and epidermal cells. Histamine is known to be released by a range of skin cells, including mast cells, keratinocytes, and melanocytes (Gilchrest, 1981; Yoshida, 2002). The time course of histamine induction and the immediate reaction observed suggest that a major portion of the histamine released was from intracellular storage granules, such as found in mast cells and basophils, although other cells may have contributed.

Histamine receptors are displayed on a wide range of resident epithelial cells, including cells of the monocyte/macrophage lineage, neutrophils, and T- and β-lymphocytes (Jutel., 2005). Many of the proinflammatory effects of histamine are mediated via the transcription factor NF-κB (Bakker, 2001). These may include expression of adhesion molecules, cytokines and chemokines, nitric oxide, and eicosanoids (Kubes and Kanwar, 1994; Saito, 1996; Kohda, 2002; Giustizieri, 2004; Jutel, 2005).

It has been constantly observed that topical or systemic ALA-PDT results in the potent recruitment and activation of neutrophils and histamine was shown to be essential for the induction of both the early-(2 hours post-treatment) and later-phase neutrophilia (8 hours post-treatment), in PDT of murine tumors (Cecic and Korbelik, 2002), and a neutrophilia appears vital for successful PDT (de Vree, 1996).
It is likely that the histamine originates from the activation/degranulation of mast cells. This is evidenced by the fact that histamine is early released following mast cell degranulation and that ALA-PDT has been shown to activate mast cells (Brooke, 2006). The known cross talk between neutrophils and mast cells largely contributes to the ALA-PDT induced inflammation.

It should be noticed that in addition to histamine, tryptase is as well a pre-formed mast cell mediator which is therefore rapidly release following mast cell activation. Tryptase is a serine protease and is the most abundant mediator stored in mast cell granules. The release of tryptase from the secretory granules is a characteristic feature of mast cell degranulation. Mast cell tryptase has an important role in inflammation. Among various physiopathological roles, tryptase activates the protease activated receptor type 2 which may result in neurogenic inflammation and pain (Oliveira, 2013). Along this line, it should be noticed as well that mast cells reside near the nerve fibers, which makes them an ideal candidate for modulating neural activity and nociception (Aich, 2015). MCs can interact with the nervous system bi-directionally, as mast cell-derived mediators such as tryptase and histamine lead to release of neuropeptides, e.g., SP and calcitonin gene-related peptide (CGRP) from the proximal nerve endings (Kleij, 2005), and subsequently SP can further activate mast cell. Increased mast cell counts in proximity of neural system (Anaf, 2006, Barbara, 2004) and abnormalities in nerve fiber structure have been correlated with nerve growth factor (NGF), a mast cell-derived and nerve fiber-derived mediator which is a bidirectional source of hyperalgesia (Watson, 2008).

Another undesirable effect of PDT treatment which is often seen after illumination is Hyperpigmentation of the skin (i.e. increase in pigmentation). Regulation of cutaneous pigmentation is dependent on several processes comprising melanin synthesis within the melanosome and efficiency of melanosomal transfer from melanocytes to keratinocytes, followed by melanosome processing in the recipient keratinocytes.

Melanocytes are cells of the follicular and interfollicular epidermis. These cells produce a specialized lysosomal related organelle referred to as melanosome. Within the melanosome, biopolymers of the pigment melanin are synthesized to give skin, as well as other tissue, its color. This melanin synthesis involves a bipartite process in which structural proteins are exported from the endoplasmic reticulum and fuse with melanosome-specific regulatory glycoproteins released in coated vesicles from the Golgi-apparatus. Melanin synthesis ensues subsequent to the sorting and trafficking of these proteins to the melanosome. Each melanocyte resides in the basal epithelial layer and, by virtue of its dendrites, interacts with approximately 36 keratinocytes. Furthermore, the amount and type of melanin produced and transferred to the keratinocytes with subsequent incorporation, aggregation and degradation influences skin complexion coloration. Hyperpigmentary disorders of the skin such as melasma, agespots or solar lentigo can result from the overproduction and accumulation of melanin. As such, depigmenting agents have potent effects by acting on one or more steps in the melanogenic pathway, melanosome transfer or post-transfer pigment processing and degradation.

Tyrosinase (E.C. 1.14.18.1) is an oxidase that is the rate-limiting enzyme for controlling the production of melanin. The enzyme is mainly involved in two distinct reactions of melanin synthesis; firstly, the hydroxylation of a monophenol and secondly, the conversion of an o-diphenol to the corresponding o-quinone.

It is an enzyme with two cupper ions coordinated by three histidines is a bifunctional enzyme that catalyses both the hydroxylation of tyrosine to L-DOPA and the consequent oxidation of the resulting catechol-containing species to an o-quinone.

The biosynthesis of the two major forms of melanin, black/brown eumelanin and yellow/red pheomelanin is initially catalyzed by tyrosinase. Specifically, the enzyme catalyzes the hydroxylation of the monophenol I-tyrosine to the o-diphenol 3,4-dihydroxyphenylalanine (DOPA) and the oxidation of DOPA to the o-quinone DOPAquinone.

The pharmaceutical compositions of the present invention are preferably administered or applied topically to a subject in need thereof, for relief of pain, and other photodynamic therapy side effects such as (hyper) pigmentation and inflammation.

Preferably, the pharmaceutical compositions are administrated topically in a separate manner (in a chronically staggered manner, especially a sequence-specific manner). As used herein, "separate" or "separately" encompasses sequential or subsequent administration and refers to the administration of the first pharmaceutical composition, followed by a time period of discontinuance, which is then followed, by the administration of the second pharmaceutical composition.

Alternatively, the first pharmaceutical composition can be administered without subsequent administration of the second pharmaceutical composition. Alternatively, also, the second pharmaceutical composition can be administered without being preceded by the administration of the first pharmaceutical composition.

Usually, the first pharmaceutical composition is administered before, during and/or after Photodynamic therapy administration. Preferably the first pharmaceutical composition is administered before Photodynamic therapy administration, i.e. skin light administration. The time period between the administration of the first pharmaceutical composition and skin light irradiation may be comprised between about 1 hour and about 5 minutes preferably between about 20 minutes and about 5 minutes, most preferably between about 10 minutes and about 5 minutes.

Usually also, the second pharmaceutical composition is administered after Photodynamic therapy administration. The time period between the end of light irradiation and the topical administration of the second pharmaceutical composition may be comprised between about 1 min and about 1 hour, preferably between about 1 minute and about 30 minutes, most preferably between about 1 minute and about 10 minutes. The second pharmaceutical composition will be further administered, once or twice a day for a period comprised between about 1 day and about 15 days, preferably between about 5 days and about 10 days, most preferably about 7 days following the day of light irradiation.

Generally, the first pharmaceutical composition will be applied prior the light irradiation and is intended to limit acute inflammation and pain during illumination whereas the second pharmaceutical composition will be applied after said light irradiation to limit both recurrent inflammation post irradiation and the undesirable pigmentation.

Usually, the first and/or second pharmaceutical composition(s) of the composition combination of the invention further include(s) at least one pharmaceutically carrier or excipient selected from the group comprising surfactants, pigments, stabilizers, emollients, humectants, vectors or a mixture thereof.

As used herein, "pharmaceutically acceptable carrier or excipient" refers to an excipient or carrier that does not produce an adverse, allergic or other reaction when administered to an animal, preferably a human.

Preferably, when used, the vectors can be selected from any group known in the art, e.g. from the group comprising liposomes, polyplexes, lipoplexes, and squalene.

As disclosed herein, the first pharmaceutical composition of the composition combination of the invention includes a therapeutically effective amount of one or more compound(s) or agent(s) having analgesic properties and/or one or more oxy-radical scavenger(s) to provide inflammation relief and resulting pain relief during illumination.

In one aspect, the first pharmaceutical composition of the composition combination of the invention also includes a therapeutically effective amount of one or more photosensitizer (PS) such as those chosen from the group of molecules called photo-activable porphyrins (PaPs) such as protoporphyrin IX (PpIX) or a precursor thereof. Most preferred protoporphyrin IX (PpIX) are selected from the group comprising topical 5-ALA, methyl-ALA (MAL), and other intermediate photosensitizing porphyrins.

The analgesic compound or agent of the invention can be selected among the non-limiting group of tyrosinase inhibitor, terpene alcohol, flavonoid, tryptase inhibitor, oxy radical scavenger(s), mast cell activator inhibitor and combinations of one or more thereof. Additionally, one or more compound(s) or agent(s) of these groups can also exhibit anti-inflammatory properties.

More preferably, the analgesic compound or agent is a terpene alcohol selected from the group comprising a monoterpene alcohol, a sesquiterpene alcohol or a diterpene alcohol and combinations of one or more thereof. Most preferably, the one or more terpene alcohol is selected from the group comprising cedrenol, cedrols, geraniol, nerolidol, bisabolols, citronellol, carvacrol, nerol, terpineol, linalool, menthol, pulegol, carveol, pinocampheol, myrcenol, isopulegol, farnesol, lanceol, santalols, vetiverol, viridiflorol, valerianol, tumerols, patchoulol, occidol, nootkatol, jinkoh eremol, hanamyol, guaicol germacradienol, fokienol, eudesmols, and cadinols, an active optical or steric isomer of these compounds and combinations of one or more thereof. Most preferably, the monoterpene or sequiterpene is selected from the group comprising linalool, (-)-menthol, carvacrol and bisabolol , derivatives of one or more of these compounds and combinations of one or more thereof.

Examples of tyrosinase inhibitors include, but are not limited to, acid ascorbic (vitamin C), hydroquinone, flavonoids, monobenzylether, phenolic thioether, kojic acid, azelaic acid, gentisic acid, aloesin, hydroxystilbene, licorice extract, derivatives of one or more of these compounds and combinations of one or more thereof. Preferably, the tyrosinase inhibitor is selected form the group comprising ascorbic acid, hydroquinone, and gentisic acid and derivatives of one or more of these compounds and combinations of one or more thereof.

The flavonoid is preferably a flavonol selected among the group comprising quercetin, dihydroquercetin (DHQ), kaempferol, catechin, epicatechin, miricetin, rutin, morinand, derivatives of one or more of these compounds
and combinations of one or more thereof.

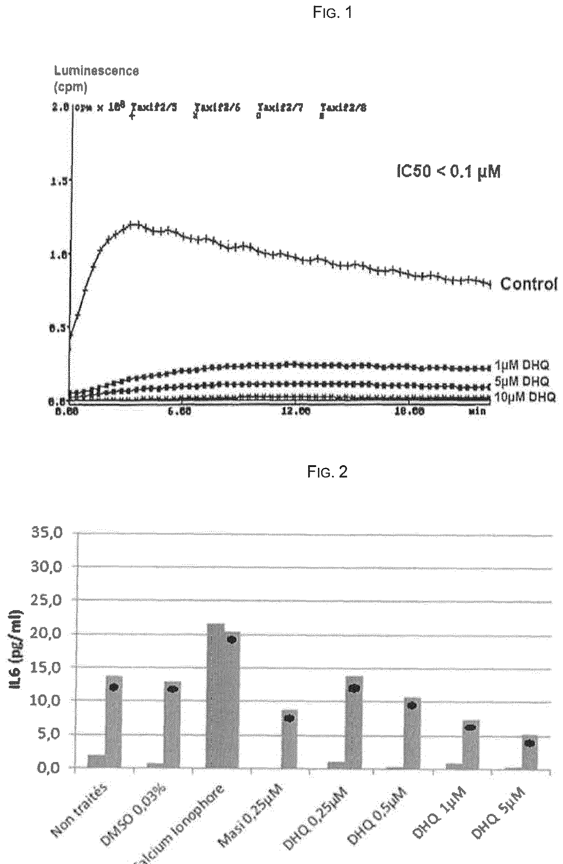

As shown in Example IV and in fig. 2, Dihydroquercetin (DHQ) is an inhibitor of IL6 release from activated mast cells thus inhibiting mast cell induced inflammation. The Inventors have also evidenced an inhibitory effect of DHQ on the reactive oxygen species (ROS) production by neutrophils (Example I, Fig. 1) as well as an inhibition of mast cells hexoamidinase release (Example III, Table 2).

The oxy-radical scavenger of the present invention can be selected among the group of tocopherols, preferably alfa-tocopherol referred to as vitamin E, or from the group of superoxide dismutase (SOD) mimics, preferably amino acids copper complexes or more preferably salicylate copper complex, derivatives of one or more of these compounds and combinations of one or more thereof.

Additionally, or alternatively the first pharmaceutical composition of the invention can further comprise a compound that facilitates the diffusion of the at least one compound(s) or agent(s) having analgesic properties and/or one or more oxy-radical scavenger(s). Preferably, this compound that facilitates the diffusion (also called skin penetration enhancer) is selected among the sesquiterpene alcohols.

In one aspect the first pharmaceutical composition of the composition combination of the invention comprises:
- from about 0.01% to about 10% in weight of the total weight of the first pharmaceutical composition of the at least one compound having analgesic properties, preferably from about 0.1% to about 5%, more preferably from about 1% to about 2%,
- from about 0.01% to about 10% in weight of the total weight of the first pharmaceutical composition of the at least one oxy-radical scavenger, preferably from about 0.1% to about 5%, more preferably from about 1% to about 3%,
- and at least one component selected from the group comprising surfactants, pigments, stabilizers, emollients, humectants, vectors or a mixture thereof,

The second pharmaceutical composition of the composition combination of the invention includes a therapeutically effective amount of one or more anti-inflammatory agent(s) and/or one or more anti-pigmentation compound(s). The aim of this second pharmaceutical composition of the composition combination is to prevent, limit or reduce recurrent inflammation and undesirable pigmentation following PDT illumination.

Several depigmenting agents modulate skin pigmentation by influencing the transcription and/or activity of tyrosinase as well as related melanogenic enzymes tyrosinase related protein-1 (TYRP-1), tyrosinase related protein-2 (TYRP-2). Thus the anti-pigmentation of the invention is preferably selected among the group comprising anti-tyrosinase, anti-tyrosinase related protein-1, tyrosinase related protein-2 derivatives of one or more of these compounds and combinations of one or more thereof.

Examples of tyrosinase inhibitors include, but are not limited to, acid ascorbic (vitamin C), hydroquinone, flavonoids, monobenzylether, phenolic thioether, kojic acid, azelaic acid, gentisic acid, aloesin, hydroxystilbene, licorice extract, oxyresveratrol, derivatives of one or more of these compounds
and combinations of one or more thereof. Preferably, the tyrosinase inhibitor exhibit anti-pigmentation properties is selected from the group comprising ascorbic acid, hydroquinone, oxyresveratrol, hydroquinone and (alpha ceto)flavonoids and gentisic acid and derivatives of one or more of these compounds
and combinations of one or more thereof.

Additionally, or alternatively to the compound(s) having tyrosinase inhibitor activity, the second pharmaceutical composition can further comprise one or more agent(s) or compound(s) inhibiting melanosome transfer that also can influence and modulate skin pigmentation. These agents or compounds inhibiting melanosome transfer will be selected from the group comprising Centaureidin, Methylophiopogonanone B, Niacinamide, PAR-2 Inhibitors, Lectins and Neoglycoproteins and derivatives of one or more of these compounds
and combinations of one or more thereof.

Additionally, or alternatively the second pharmaceutical composition of the invention can further comprise one or more depigmenting agents or compounds inhibiting tyrosinase related protein-1 (TYRP-1) and/or tyrosinase related protein-2 (TYRP-2) and combinations of one or more thereof.

The anti-inflammatory compound or agent of the invention can be selected among the non-limiting group of tyrosinase inhibitor, terpene alcohol, flavonoid, tryptase inhibitor, singlet oxygen quencher(s), oxy radical scavenger(s), mast cell activator inhibitor and combinations of one or more thereof. Preferably, the anti-inflammatory compound or agent is a flanoids, most preferably a flavonol selected among the group comprising quercetin, dihydroquercetin (DHQ), kaempferol, catechin, epicatechin, miricetin, rutin, morinand, derivatives of one or more of these compounds and combinations of one or more thereof. Even more preferably, the flavonol is dihydroquercetin (DHQ).

Additionally, or alternatively the second pharmaceutical composition of the invention can further comprise a compound that facilitates the diffusion of the one or more anti-inflammatory agent(s) and/or the one or more anti-pigmentation compound(s). Preferably, the compound that facilitates the diffusion (also called skin penetration enhancer) is selected among the sesquiterpene alcohols.

Typically, the second pharmaceutical composition of the composition combination of the invention comprises:
- from about 0.01% to about 10% in weight of the total weight of the second pharmaceutical composition of the at least one tyrosinase inhibitor, preferably from about 0.1% to about 5%, more preferably from about 1% to about 4%,
- from about 0.01% to about 10% in weight of the total weight of the second pharmaceutical composition of the at least one anti-inflammatory agent, preferably from about 0.1% to about 5%, more preferably from about 0.5% to about 2%,
- from about 0.01% to about 10% in weight of the total weight of the second pharmaceutical composition of the at least one terpene alcohol, preferably from about 0.1% to about 5%, more preferably from about 0.5% to about 2%,
and at least one component selected from the group comprising surfactants, pigments, stabilizers, emollients, humectants, vectors or a mixture thereof.

Referring in more details to the examples, the first pharmaceutical composition of the combination composition of the invention will independently be chosen among e.g. formulation 1.1, I. 2 and I. 3 and the second pharmaceutical composition will independently be chosen among, e.g. formulation II. 1, II. 2 and II.3. Any combinations between a formulation I (I.1, I.2, I.3) and a formulation II (II. 1, II. 2 and II.3) is envisioned.

Examples of Formulation I (Photooxyl I)
- Formulation I.1 (Vitamin C (3%), Linalool (1%), Bisabolol (1%))
- Formulation 1.2 (Hydroquinone, Vitamin E, Linalool (1%), Bisabolol (1%))
- Formulation 1.3 (Gentisic acid, Vitamin E, Linalool (1%), Bisabolol (1%))

Examples of Formulation II (Photooxyl II)
- Formulation 11.1 (Vitamin C (3%), Dihydroquercetin (2%), Oxyresveratrol (1%), Bisabolol (1%))
- Formulation II.2 (Vitamin E (3%), Dihydroquercetin (2%), Oxyresveratrol (1%), Bisabolol (1%))
- Formulation II.3 (Vitamin C (3%), Dihydroquercetin (2%), Oxyresveratrol (1%))

**Linalool,** 3, 7-Dimethyl-1, 6-octadien-3-ol is found in allspice. 3, 7-Dimethyl-1, 6-octadien-3-ol is a flavouring agent. 3, 7-Dimethyl-1, 6-octadien-3-ol is widespread natural occurrence as the optically active and racemic forms in over 200 essential oils. Also present in numerous fruits. Linalool is a naturally occurring terpene alcohol chemical found in many flowers and spice plants with many commercial applications, the majority of which are based on its pleasant scent (floral, with a touch of spiciness).

**Bisabolol** (also named **α-Bisabolol**) is a naturally occurring sesquiterpene alcohol which was first isolated from Matricaria chamomilla (Asteraceae) in the twentieth century and has since been identified in other aromatic plants such as Eremanthus erythropappus, Smyrniopsis aucheri and Vanillosmopsis species. Recently, α-bisabolol was identified as a major constituent of Salvia runcinata essential oil, a plant indigenous to South Africa.

This compound also exhibits several other pharmacological properties such as anti-inflammatory and analgesic properties. Referring in more details to example II and in particular to Table 1, it has been shown that Bisabolol is able to inhibit the release of polymorphonuclear neutrophils myeloperoxidase (MPO) thus blocking the recruitment and activation of polymorphonuclear neutrophils (PMNs) as well as the release of histamine from these PMNs cells.

**α-Tocopherol (vitamin E)** is a major biological lipid-soluble antioxidant. It performs its functions as antioxidant in connection with the glutathione peroxidase pathway and it protects cell membranes from oxidation by reacting with lipid radicals produced in the lipid peroxidation chain reaction. This process would remove the reactive free radical intermediates and prevent the oxidation chain reaction from continuing. The oxidized α-tocopheroxyl radicals produced in this process may be recycled back to the native reduced form through reduction by other antioxidants, such as ascorbate, retinol or ubiquinol as wells as by Al a process which support some claims of the present composition.

**Dihydroquercetin (DHQ)** 3,3',4',5,7-Pentahydroxyflavone dihydrate, 2-(3,4-Dihydroxyphenyl)-3,5,7-trihydroxy-4H-1-benzopyran-4-one dihydrate. Formula: C15H10O7·2H2O, molecular weight 338.27, referred to as Taxifolin is a natural compound of the flavonoid family characterized by a great chemical stability with conserved significant biological and pharmacological properties. In 1958 the Journal of the American Pharmaceutical Association published research establishing its safety and by the mid-1960s DHQ was being widely investigated for use as a natural preservative in all kinds of foods. DHQ is extracted from a type of larch wood and has been marketed in Russia and the US for 15-20 years as a food supplement.

DHQ, is an anti-inflammatory agent which is one of the most efficient antioxidant by scavenging oxy-radicals and singlet oxygen.

As shown in Example IV and in fig. 2, DHQ is an inhibitor of IL6 release from activated mast cells thus inhibiting mast cell induced inflammation.

The Inventors have also evidenced an inhibitory effect of DHQ on the reactive oxygen species (ROS) production by neutrophils (Example I, Fig. 1) as well as an inhibition of mast cells hexoamidinase release (Example III, Table 2).

**Oxyresveratrol** is a naturally occurring analog of resveratrol found in mulberry wood. It effectively scavenges H2O2, NO (IC50 = 45.3 µM), and the artificial free radical 2,2-diphenyl-I-picrylhydrazyl (IC50 = 28.9 µM).2 At 10 mg/kg, oxyresveratrol acts as a neuroprotectant, reducing brain infarct volume and reducing cytochrome c release and caspase-3 activation in an in vivo model of stroke. Oxyresveratrol also has depigmenting effects by effectively inhibiting tyrosinase activity, which catalyzes the rate-limiting step in synthesizing melanin pigments (IC50s = 1.2 and 52.7 µM in mushroom and mouse melanoma B-16 cells, respectively). It is 32-fold more potent than kojic acid, a depigmenting agent used in cosmetic materials with skin-whitening effects and medical agents used to treat hyperpigmentation disorders.

The first and/or second pharmaceutical composition(s) of the composition combination of the invention is/are preferably under a form suitable for topical application such as a cream, a gel, an ointment, a solution, an emulsion, a mask, a milk, a lotion, a serum, a paste, a foam or a suspension, preferably a cream. The first and the second pharmaceutical compositions can be in the same form or in different forms suitable for topical application (e.g. cream and gel, gel and emulsion, cream and patch, etc..).

Alternatively, the second pharmaceutical composition of the composition combination of the invention is present in or on a topical drug delivery patch.

The first and/or second pharmaceutical composition(s) of the composition combination of the invention is/are preferably under the form of an oil-in-water emulsion. Most preferably, the oily and aqueous phases are in an oily phase/aqueous phase weight ratio of from about 95/5 to about 5/95 and preferably from about 30/70 to about 80/20.

Another aspect of the present invention is to provide a kit comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
optionally with instructions to use.

A further aspect of the present invention is to provide a method for the treatment and/or prevention of Photodynamic therapy side effects comprising separately administering in a subject in need thereof
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
wherein administration is a topical administration.

Also comprised in the present invention is a composition combination comprising
i) a first composition comprising a therapeutically effective amount of one or more analgesic agent(s) and/or one or more oxy-radical scavenger(s), and
ii) a second composition comprising a therapeutically effective amount of one or more anti-inflammatory agent(s) and/or one or more anti-pigmentation compound(s).

Further comprised in the present invention is a cosmetic composition combination comprising
i) the first composition of the invention, and
ii) the second composition the invention.

Hereinafter, the present invention will be explained in more detail with reference to the following preferred embodiment and experimental examples. It should be noted that the following preferred embodiment and experiment examples serve as examples of the present invention, and thus claims of the present invention are not limited thereto.

### EXAMPLES

### Example I

### Dihydroquercetin mediated inhibition of polymorphonuclear neutrophils oxidative burst (Fig. 1)

Inhibitory effect of DHQ on the reactive oxygen species (ROS) production resulting from the neutrophils activation by 100 ng/ml PMA. ROS production is measured using a chemiluminescence standard procedure: briefly, one hundred microliters of human neutrophils (4 × 106/mL) were primed with TNF-α at 37°C for 25 minutes. 100 µL luminol (1 µM final concentration) and HRP (62.5 U/mL final concentration) in HBSS were added, and 150-µL aliquots were transferred to a prewarmed 96-well luminometer plate. Light emission was recorded by a Berthold MicroLumat Plus luminometer (Berthold Technologies, Hartfordshire, United Kingdom) (data output is in relative light units per second)

### Example II

### Bisabolol mediated inhibition of polymorphonuclear neutrophils myeloperoxidase release (Table 1).

Neutrophils were stimulated with formyl-methionyl-leucyl-phenylalanine for 45 minutes at 37° C. MPO released from neutrophils were assayed by using an MPO enzyme immunoassay.

**Table 1: Bisabolol-mediated Inhibition of neutrophils degranulation assessed by the release of myeloperoxidase (MPO). human neutrophils (4 × 10₆/mL).**

| Bisabolol Concentrations (µM) | MPO release (U/mL) |
|---|---|
| 0.00 | 800 |
| 20 | 810 |
| 40 | 500 |
| 100 | 250 |
| 200 | 248 |

### Example III

### Quercetin and Dihydroquercetin (DHQ) mediated inhibition of mast cells hexoamidinase release (Table 2).

Degranulation was induced by the addition of IgE followed by the addition of anti-IgE Beta-hexosaminidase was measured 1 hour after IgE stimulation. Reference molecule was cromoglycate.

**Table 2: Effect of DHQ on mast cell degranulation as estimated by beta-hexosaminidase release.**

| Concentrations | Control without IgE | Reference molecule | DHQ |
|---|---|---|---|
| 0.00 | 0.130 | 0.79 | 0.82 |
| 10-5 M | 0.135 | 0.48 | 0.52 |
| 10-4 M | 0.132 | 0.34 | 0.28 |

### Example IV

### Dihydroquercetin (DHQ) mediated inhibition of IL6 release from activated mast cells (Fig. 2)

Mast cells were obtained from CD34+ progenitors cultivated during 8 weeks in the presence of stem cell factor and IL3. Some mast cells (light grey with spots) were primed during 5 days by IL4 and IgE. Primed mast cells were activated by anti IgE and IL6 release is measured 6 hours following the activation. Masitinib (Masi) a c-kit receptor inhibitor is taken as positive control. Dark grey corresponds to the basal release of IL6 in the absence of mast cells activation. Maximum release of resting mast cells were obtained by the use of a calcium ionophore.

### Example V

### Examples of Formulation I (Photooxyl I):

Should be used during the light irradiation.
Objective: limitation of acute inflammation and pain during illumination
Contains as active ingredients (%):

### • Formulation 1.1

Vitamin C (3%)
Linalool (1%)
Bisabolol (1%)

### • Formulation I.2

Hydroquinone
Vitamin E
Linalool (1%)
Bisabolol (1%)

### • Formulation I.3

Gentisic acid
Vitamin E
Linalool (1%)
Bisabolol (1%)

### Examples of Formulation II (Photooxyl II)

Should be used after light irradiation for one week.

Objective: limitation of recurrent inflammation post irradiation, limitation of the undesirable pigmentation.

Contains as active ingredients (%):

### • Formulation II.1

Vitamin C (3%)
Dihydroquercetin (2%)
Oxyresveratrol (1%)
Bisabolol (1%)

### • Formulation II.2

Vitamin E (3%)
Dihydroquercetin (2%)
Oxyresveratrol (1%)
Bisabolol (1%)

### • Formulation II.3

Vitamin C (3%)
Dihydroquercetin (2%)
Oxyresveratrol (1%)

### Description of the formulation components:

A cosmetically acceptable cream base (bio grade).
Example: water based O/W
AQUA (ultra purified)
SWEET ALMOND OIL
CAPRYLIC/CAPRIC TRIGLYCERIDE
OLUS OIL
BUTYROSPERMUM PARKII BUTTER
DICAPRYLYL ETHER
GLYCERIN
PROPANEDIOL
ALCOHOL DENAT
SORBITOL
CETEARYL ALCOHOL
GLYCERYL STEARATE
GLYCERYL STEARATE CITRATE
POLYGLYCERYL-3 METHYLGLUCOSE DISTEARATE
XANTHAN GUM
SODIUM DEHYDROACETATE
SODIUM BENZOATE
PHENOXYETHANOL
CITRIC ACID

### Example VI - Effect of PHOTOOXYL protocol on the extent of PDT-induced skin inflammation

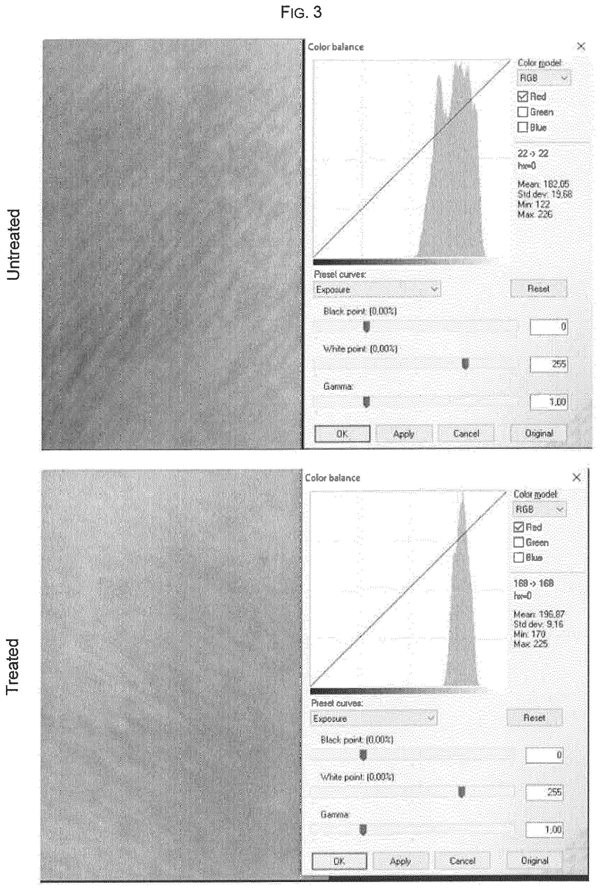

Fig. 3 shows a comparative evaluation of skin erythema 8 days after the PDT irradiation between treated and non-treated skin. (Healthy volunteer 1)

### Example VII Effect of PHOTOOXYL protocol on the extent of PDT-induced skin inflammation

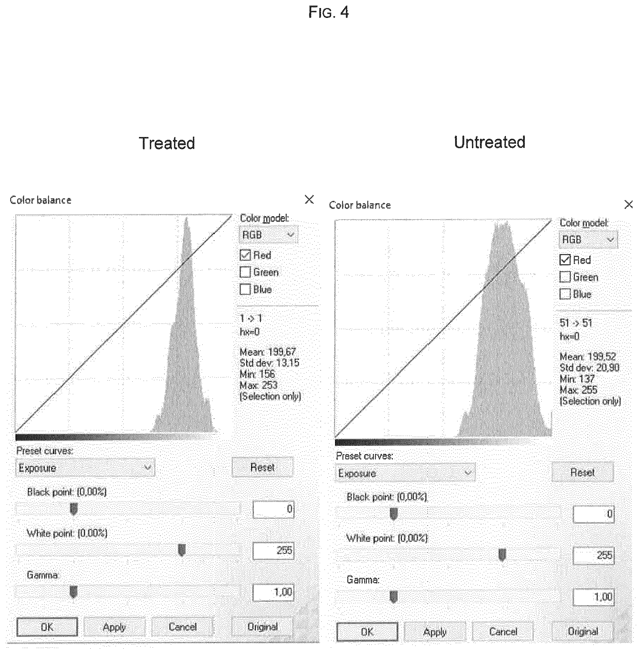

Fig. 4 shows a comparative evaluation of skin erythema 2, 3 and 4 days after the PDT irradiation between treated and non-treated skin. (Healthy volunteer 2)

In treated experiment (left arm), scraped skin area was treated with formulation I 15 minutes before irradiation and irradiated skin area was further treated daily with topical formulation II. Estimation of inflammation/pigmentation was performed on day 2, 3, 4 following PDT irradiation. Image analyzing has been done on pictures recorded on day 4.
Formulation I.1 composition: ascorbic acid 3%, linalool 1%, bisabolol 1%
Formulation II.3 composition: ascorbic acid 3%, dihydroquercetin 2%, oxyresveratrol 1%

### REFERENCES

Aich Anupam, Lawrence B. Afrin and Kalpna Gupta, Mast Cell-Mediated Mechanisms of Nociception, Int. J. Mol. Sci. 2015, 16, 29069-29092
Anaf, V.; Chapron, C.; el Nakadi, I.; de Moor, V.; Simonart, T.; Noel, J.C. Pain, mast cells, and nerves in peritoneal, ovarian, and deep infiltrating endometriosis. Fertil. Steril. 2006, 86, 1336-1343.
Barbara, G., Stanghellini, V., de Giorgio, R., Cremon, C.; Cottrell, G.S.; Santini, D.; Pasquinelli, G.; Morselli-Labate, A.M.; Grady, E.F.; Bunnett, N.W.; et al. Activated mast cells in proximity to colonic nerves correlate with abdominal pain in irritable bowel syndrome. Gastroenterology 2004, 126, 693-702.
Brooke Rebecca C.C. , Animesh Sinha, Meneka K. Sidhu, Rachel E.B. Watson, Martin K. hurch2, Peter S. Friedmann, Geraldine F. Clough, Lesley E. Rhodes, Histamine Is Released following Aminolevulinic Acid-Photodynamic Therapy of Human Skin and Mediates an Aminolevulinic Acid Dose-Related Immediate Inflammatory Response, Journal of Investigative Dermatology Volume 126, Issue 10, October 2006, Pages 2296-2301
De Vree, WJ., M.C. Essers, H.S. de Bruijn, W.M. Star, J.F. Koster, W. Sluiter Evidence for an important role of neutrophils in the efficacy of photodynamic therapy in vivo Cancer Res, 56 (1996), pp. 2908-2911
Cecic,I and Korbelik M, Mediators of peripheral blood neutrophilia induced by photodynamic therapy of solid tumors Cancer Lett, 183 (2002), pp. 43-51
Gilchrest, BA, N.A. Soter, J.S. Stoff, M.C. Mihm Jr. The human sunburn reaction: histologic and biochemical studies J Am Acad Dermatol, 5 (1981), pp. 411-422
Giustizieri, M.L., C. Albanesi, J. Fluhr, P. Gisondi, J. Norgauer, G. Girolomoni H1 histamine receptor mediates inflammatory responses in human keratinocytes J Allergy Clin Immunol, 114 (2004), pp. 1176-1182
Jutel, M., K. Blaser, C.A. Akdis Histamine in allergic inflammation and immune modulation Int Arch Allergy Immunol, 137 (2005), pp. 82-92
Kennedy J.C., Pottier RH. Endogenous protoporphyrin IX, a clinically useful photosensitizer for photodynamic therapy. J Photochem Photobiol B Biol. 1992; 14:275-92.
Kleij, H.P.; Bienenstock, J. Significance of conversation between mast cells and nerves. Allergy Asthma Clin. Immunol. 2005, 1, 65-80.
Kohda, F., T. Koga, H. Uchi, K. Urabe, M. Furue Histamine-induced IL-6 and IL-8 production are differentially modulated by IFN-gamma and IL-4 in human keratinocytes J Dermatol Sci, 28 (2002), pp. 34-41
Oliveira SM, Silva CR, Ferreira J. Critical role of protease-activated receptor 2 activation by mast cell tryptase in the development of postoperative pain. Anesthesiology. 2013 Mar; 118(3):679-90.
Orenstein A, Kostenich G, Tsur H, et al. Temperature monitoring during photodynamic therapy of skin tumors with topical 5-aminolevulinic acid application. Cancer Lett. 1995; 93:227-32.
Watson, J.J.; Allen, S.J.; Dawbarn, D. Targeting nerve growth factor in pain: What is the therapeutic potential? BioDrugs 2008, 22, 349-359.
Yoshida, M, S. Hirotsu, M. Nakahara, H. Uchiwa, Y. Tomita Histamine is involved in ultraviolet B-induced pigmentation of guinea pig skin J Invest Dermatol, 118 (2002), pp. 255-260 (abstr.)

## Claims

1. A composition combination for use in the topical treatment and/or prevention of Photodynamic therapy side effect(s), said pharmaceutical combination comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
wherein said first and second pharmaceutical compositions are administered separately in a subject in need thereof.

2. The composition combination for use of claim 1, wherein said first pharmaceutical composition is administered before, during and/or after Photodynamic therapy administration and said second pharmaceutical composition is administered after Photodynamic therapy administration.

3. The composition combination for use of anyone of the preceding claims, wherein the Photodynamic therapy side effect(s) is selected from the group comprising Photodynamic therapy-induced skin inflammation, pain, pigmentation and combinations of one or more thereof.

4. The composition combination for use of anyone of the preceding claims, wherein the one or more analgesic agent(s) is selected from the group comprising tyrosinase inhibitor, terpene alcohol, flavonoid, tryptase inhibitor, oxy radical scavengers, mast cell activator inhibitor and combinations of one or more thereof.

5. The composition combination for use of anyone of the preceding claims, wherein the one or more anti-inflammatory agent(s) is selected from the group comprising a tyrosinase inhibitor, terpene alcohol, flavonoid, tryptase inhibitor, singlet oxygen quenchers, oxy radical scavengers, mast cell activator inhibitor
and combinations of one or more thereof.

6. The composition combination for use of claim 5, wherein said tyrosinase inhibitor is selected from the group comprising acid ascorbic (vitamin C), hydroquinone, flavonoids, monobenzylether, phenolic thioether, kojic acid, azelaic acid, gentisic acid, aloesin, hydroxystilbene, licorice extract, derivatives of one or more of these compounds and combinations of one or more thereof.

7. The composition combination for use of claim 5, wherein said terpene alcohol is a monoterpene alcohol, a sesquiterpene alcohol or a diterpene alcohol or combinations of one or more thereof.

8. The composition combination for use of claim 7, wherein said terpene alcohol is selected from the group comprising a cedrenol, cedrol, geraniol, nerolidol, bisabolol, citronellol, nerol, terpineol, linalool, menthol, pulegol, carveol, pinocampheol, myrcenol, isopulegol, farnesol, lanceol, santalol, vetiverol, viridiflorol, valerianol, tumerol, patchoulol, occidol, nootkatol, jinkoh eremol, hanamyol, guaicol germacradienol, fokienol, eudesmol, and cadinol, or an optical or steric isomer of these compounds and combinations of one or more thereof.

9. The composition combination for use of anyone of the preceding claims, wherein the subject in need thereof is a subject undergoing, or will undergo, a PDT treatment.

10. The composition combination for use of anyone of the preceding claims, wherein the first pharmaceutical composition comprises a therapeutically effective amount of linalol, vitamin C, vitamin E and bisabolol and/or derivatives of one or more of these compounds.

11. The composition combination for use of anyone of the preceding claims, wherein the second pharmaceutical composition comprises a therapeutically effective amount of dihydroquercetin, vitamin C, oxyresveratrol and bisabolol and/or derivatives of one or more of these compounds.

12. The composition combination for use of anyone of the preceding claims, wherein the first and/or second pharmaceutical composition(s) further comprise(s) at least one component selected from the group comprising surfactants, pigments, stabilizers, emollients, humectants, vectors or a mixture thereof.

13. The composition combination for use of claim 12 wherein the vectors are selected from the group comprising liposomes, polyplexes, lipoplexes, and squalene.

14. The composition combination for use of anyone of the preceding claims, wherein the first and/or second pharmaceutical compositions is/are in the form of a cream, a gel, an ointment, a solution, an emulsion, a mask, a milk, a lotion, a serum, a paste, a foam or a suspension, preferably a cream.

15. The composition combination for use of anyone of the preceding claims, wherein said first and/or second pharmaceutical composition(s) is/are in the form of an oil-in-water emulsion.

16. The composition combination for use of claim 15, wherein the oily and aqueous phases is in an oily phase/aqueous phase weight ratio of from 95/5 to 5/95 and preferably from 30/70 to 80/20.

17. A kit comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
optionally with instructions to use.

18. A composition combination comprising
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s).

19. A method for the treatment and/or prevention of Photodynamic therapy side effects comprising separately administering in a subject in need thereof
i) a first pharmaceutical composition comprising a therapeutically effective amount of one or more analgesic agent(s) and/or one or more oxy-radical scavenger(s),
ii) a second pharmaceutical composition comprising a therapeutically effective amount of one or more anti-inflammatory agent(s) and/or one or more anti-pigmentation compound(s),
wherein administration is a topical administration.
